Europäisches Patentamt

European Patent Office

Office européen des brevets

(11) Publication number: **0 645 452 A1**

## EUROPEAN PATENT APPLICATION

(21) Application number: **94113232.6**

(22) Date of filing: **10.10.86**

(51) Int. Cl.⁶: **C12N 15/24**, C07K 14/54,
C12N 1/21, A61K 38/20

This application was filed on 24.08.1994 as a divisional application to the application mentioned under INID code 60

(30) Priority: **14.10.85 IL 7671485**
**08.05.86 US 860883**

(43) Date of publication of application:
**29.03.95 Bulletin 95/13**

(60) Publication number of the earlier application in accordance with Art.76 EPC: **0 536 520**

(84) Designated Contracting States:
**BE CH DE ES FR GB GR IT LI NL SE**

(71) Applicant: **YEDA RESEARCH AND DEVELOPMENT CO. LTD.**
**P.O. Box 95**
**Rehovot 76100 (IL)**

(72) Inventor: **Revel, Michael**
**Beth Brasil 5**
**Weizmann Inst. of Science Rehovot (IL)**
Inventor: **Zilberstein, Asher**
**Levin Epstein 50**
**Rehovot (IL)**

(74) Representative: **Patentanwälte Grünecker,**
**Kinkeldey, Stockmair & Partner**
**Maximilianstrasse 58**
**D-80538 München (DE)**

(54) Human interferon-beta 2A and interfeon-beta 2B, vectors containing genes coding for said interferons, cell lines producing same and use of said interferons as pharmaceuticals.

(57) Human interferon-beta$_{2A}$ and interferon-beta$_{2B}$ are produced in purified form by recombinant DNA techniques. Two separate human genes have been identified which code for the production of IFN-$\beta_{2A}$ and IFN-$\beta_{2B}$, respectively. The sequence of IFN-$\beta_{2A}$ cDNA is established. These genes and cDNA have been cloned into mammalian cells with an SV40 early promoter sequence and such genomic clones are capable of producing IFN-$\beta_{2A}$ and IFN-$\beta_{2B}$. The antiviral activity of such recombinant IFN-$\beta_{2A}$ and IFN-$\beta_{2B}$ is demonstrated as well as other biological activity identifying them as human interferons. It has been shown that IFN-$\beta_2$ secretion is induced in human cells by growth-stimulatory cytokines.

IFN-$\beta_{2A}$ may be used for preparing pharmaceutical compositions useful in the fields of inflammation,, acute phase response or regulation of cell proliferation. Further uses are suggested.

EP 0 645 452 A1

Rank Xerox (UK) Business Services
(3.10/3.09/3.3.4)

FIG.1

Interferon is an important antiviral and antitumor protein produced by the human body. Because of its species specificity, clinical use of interferon requires human interferon. Based on their neutralization by antibodies three types of interferon were defined which include interferon-alpha, produced mainly by leucocytes, interferon-beta, produced mainly by fibroblasts, and interferon-gamma, produced mainly by T-lymphocytes.

The major species of interferon produced by human fibroblasts in response to ds RNA is the 20 Kd glycoprotein IFN-$\beta_1$, encoded by a 0.9 kb RNA which is transcribed from an intron-less gene on chromosome 9. However, additional mRNAs which yield IFN activity when microinjected into frog oocytes have been observed in human fibroblasts induced by poly (rI)(rC) and by a sequential cycloheximide (CHX)-actinomycin D treatment. While cloning the IFN-$\beta_1$ cDNA, cDNA clones of another co-induced 1.3 kb RNA have been isolated which in reticulocyte lysates code for a 23-26 Kd polypeptide and in oocytes produce human IFN antiviral activity (J. Weissenbach et al. 1980 Proc. Natl. Acad. Sci. USA 77:7152-7156; British patent 2 063 882.)

Since this activity was inhibited by anti-IFN-$\beta_1$ antibodies, it was designated IFN-$\beta_2$. The protein product of IFN-$\beta_2$ RNA is immunologically distinct from IFN-$\beta_1$, although the biological activities of the two proteins are cross-neutralized by the same antibodies. The IFN-$\beta_2$ 1.3 kb RNA clearly originates from another gene than IFN-$\beta_1$, since IFN-$\beta_2$ RNA is formed in mouse-human hybrids lacking chromosome 9 (U. Nir (1984) Ph.D. Thesis, Weizmann Institute of Science, Rehovot, Israel).

In the German patent application P 30 43 981 a process is disclosed for the preparation of interferon-$\beta_2$ in purified form. Said interferon-$\beta_2$ is produced by isolating DNA containing the nucleotide sequence coding for interferon-$\beta_2$ in human cells after cultivating those cells which are able to produce interferon-$\beta_2$ when exposed to an inducer of interferon. mRNA from said induced cells is extracted and purified. Afterwards, the mRNA is transcribed into DNA and the DNA is cloned in a suitable vector.

It could be demonstrated that there is a second gene encoding IFN-$\beta_2$, which, when isolated and transfected into hamster or mouse cells produces human-specific IFN activity after induction by ds RNA and cycloheximide. The IFN produced has the properties ascribed to IFN-$\beta_2$ but does not have identical amino acid sequences, the proteins produced by the two genes are denominated IFN-$\beta_{2A}$ and IFN-$\beta_{2B}$ respectively.

Accordingly, it is an object of the present invention to isolate biologically active human IFN-$\beta_{2A}$ and IFN-$\beta_{2B}$.

It is another object of the present invention to enable the production of biologically active human IFN-$\beta_{2A}$ and IFN-$\beta_{2B}$.

It is yet another object of the present invention to prepare cDNA which codes for numan IFN-$\beta_2$, and, more particularly, cDNA which codes for human IFN-$\beta_{2A}$ and cDNA which codes for human IFN-$\beta_{2B}$.

It is a further object of the present invention to identify genes hybridizing to IFN-$\beta_2$ cDNA.

It is still another object of the present invention to identify genes hybridizing to IFN-$\beta_{2A}$ cDNA and to IFN-$\beta_{2B}$ cDNA.

It is yet a further object of the present invention to isolate genomic clones containing an IFN-$\beta_2$ gene, and, more particularly, a genomic clone containing an IFN-$\beta_{2A}$ gene and a genomic clone containing an IFN-$\beta_{2B}$ gene.

It is another object of the present invention to provide a recombinant vector containing an IFN-$\beta_{2A}$ and/or IFN-$\beta_{2B}$ gene and a promoter sequence capable of exerting transcription control over the interferon gene.

It is yet another object of the present invention to provide cells which produce biologically active human IFN-$\beta_{2A}$ and/or IFN-$\beta_{2B}$.

It is another object of the present invention to provide such cells which produce substantial quantites of such IFN-$\beta_{2A}$ and/or IFN-$\beta_{2B}$.

It is still another object of the present invention to provide a process for the production of biologically active human IFN-$\beta_{2A}$ and/or IFN-$\beta_{2B}$ by recombinant DNA technology.

It is a further object of the present invention to provide genes which, when fused to a strong viral transcriptional promoter and transfected into hamster or mouse cells, produce substantial quantities of biologically active human IFN-$\beta_{2A}$ and/or IFN-$\beta_{2B}$.

It Is still another object of the present Invention to use IFN-$\beta_{2A}$ and/or IFN-$\beta_{2B}$ in pharmaceuticals which are useful wherever it is desired to influence cell growth and differentiation, especially during terminal differentiation of cancer cells.

A further object of the invention is the use of IFN-$\beta_{2A}$ and/or IFN-$\beta_{2B}$ to inhibit fibroblast proliferation, to prevent sclerosis after infection.

In accordance with the present invention, the existence of biologically active human interferon-beta$_2$ - (IFN-$\beta_2$) molecules has been confirmed and these molecules are produced by recombinant DNA technology. IFN-$\beta_2$ is a glycoprotein secreted by human fibroblasts which are induced by double-stranded (ds) RNA or viruses to produce interferon. IFN-$\beta_2$ amounts to about 5% of the total IFN activity produced by fibroblasts. The amino acid sequence of IFN-$\beta_2$, which was determined by cDNA sequencing, indicates only about 20% overall homology with that of the other human IFNs produced by such human cells (IFN-$\beta_1$ and IFN-$\alpha$s).

A human cDNA or gene encoding IFN-$\beta_{2A}$ may be cloned in accordance with the present invention and fused to a strong viral transcriptional promoter. Hamster cells transformed by the fused cDNA or gene produce constitutively human-specific interferon activity which inhibits viral replication and cytopathic effect and induces the proteins typical of the biological response of human cells to interferons. A second gene encoding IFN-$\beta_2$ is also disclosed which, when isolated and transfected into hamster or mouse cells, produces human-specific IFN activity after induction by ds RNA and cycloheximide. As the IFN produced by both genes have the properties ascribed to IFN-$\beta_2$, but do not have identical amino acid sequences, the proteins produced by the two genes are denominated IFN-$\beta_{2A}$ and IFN-$\beta_{2B}$ respectively.

Description of the drawings

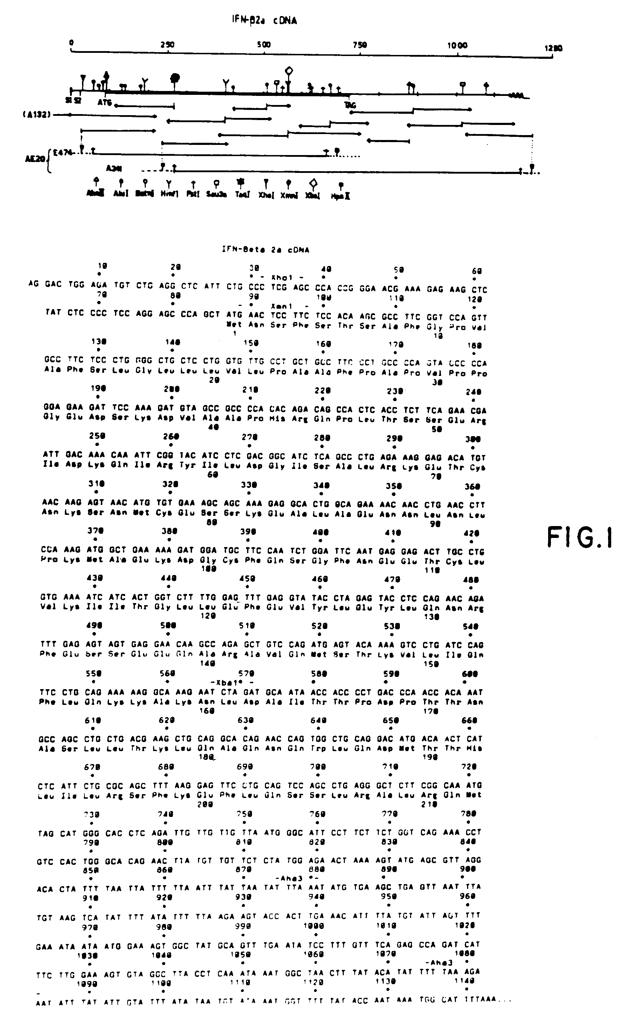

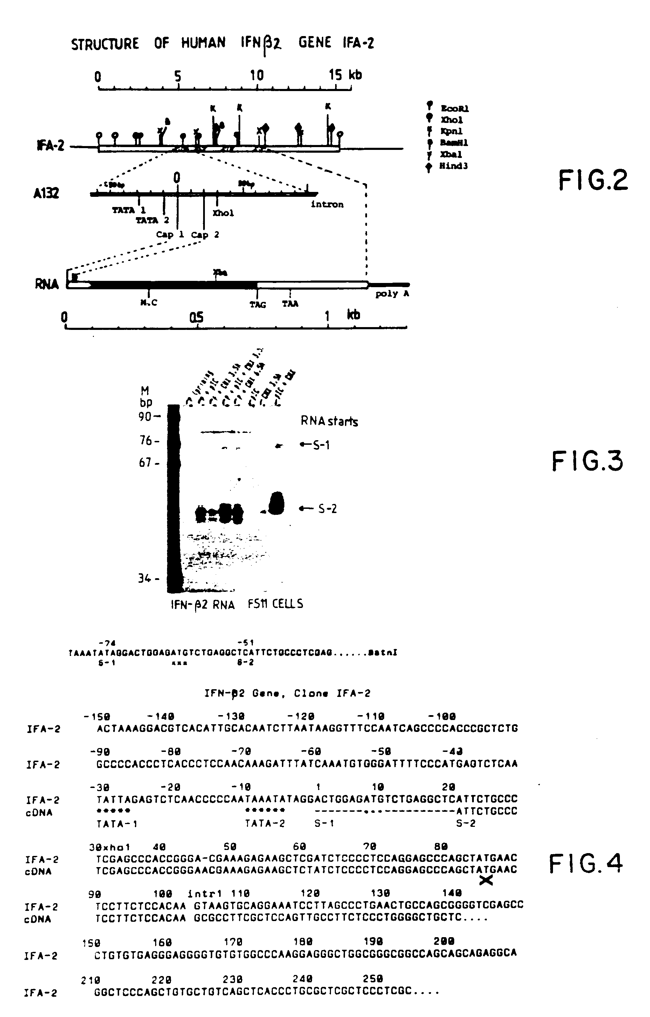

Figure 1 shows the restriction map, sequencing strategy and nucleotide sequence of IFN-$\beta_{2A}$ cDNA determined from clone AE20, formed by fusing E474 cDNA and A341 cDNA clones (Weissenbach et al. (1980), supra) by their Xba-1 site. The 5' end sequence was completed from the genomic clone IFA-2 shown in Fig. 2. Numbering begins from the S-1 start of Fig. 3 and 4. The amino acid sequence of the IFN-$\beta_{2A}$ protein is deduced.

Fire 2 shows the structure of genomic clone IFA-2 containing the IFN-$\beta_{2A}$ gene. A132 is a subclone of an EcoRI segment of IFA-2 in pBR322. Shaded areas are gene regions hybridizing to cDNA clones A341 and E474 (Weissenbach et al. (1980), supra). The positions of the two RNA starts (cap 1 and 2) and the TATA boxes are shown. In the RNA the longest open reading frame (ORF) of 212 amino acids (Fig. 1) is shown in black. M.C. indicates a second internal ATG in the same frame. No other long ORF are found in the entire IFN-$\beta_{2A}$ cDNA (Fig. 1).

Figure 3 shows the S1 nuclease analysis of 5' starts in IFN-$\beta_2$ RNA. The DNA probe, a fragment from subclone A132 of IFA-2 gene (Figure 2) labelled at BstN1 site was hybridized to total RNA from diploid fibroblasts treated (from left to right) by: priming with IFN-$\beta_1$ 200 U/ml,16 hrs; priming and poly (rI)(rC)(pIC) 50 $\mu$g/ml, 3.5 hours; priming and cycloheximide (CHX) 50 $\mu$g/ml, 3.5 hours; priming and pIC plus CHX 3.5 hours; priming and CHX for 6.5 hours; no priming and pIC 3.5 hours; same CHX 3.5 hours; same pIC plus CHX 3.5 hours. S-1 and S-2 are the signals generated by the 2 RNA starts of Figure 2.

Figure 4 shows the sequence of the promoter region of the IFA-2 gene encoding IFN-$\beta_{2A}$. The sequence of part of subclone A132 (Figure 2) is shown compared to the IFN-$\beta_2$ cDNA. S1 and S2 are the 2 RNA starts determined in Figure 3. The initiator ATG is marked by X. The first intron starts at intr 1.

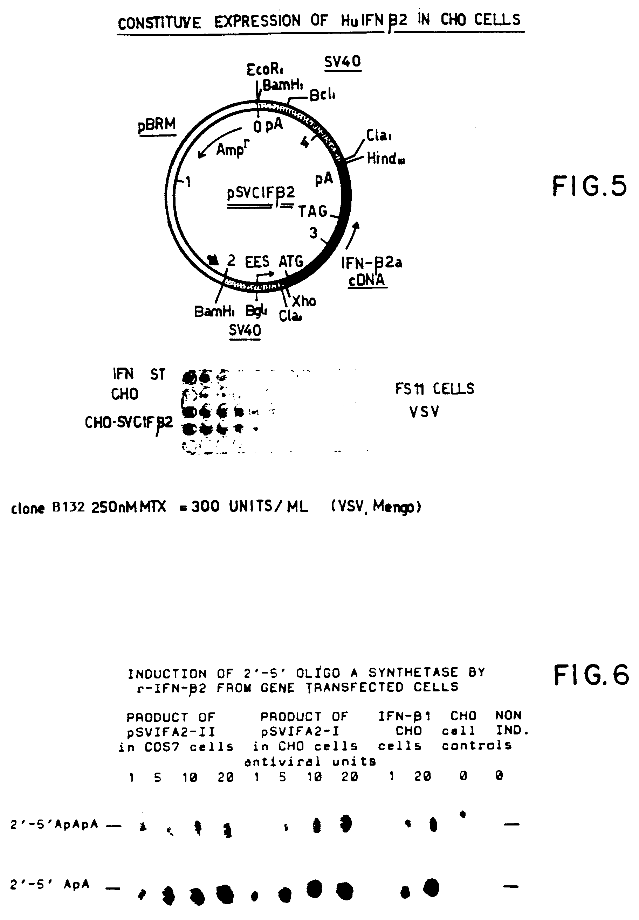

Figure 5 illustrates the fusion of IFN-$\beta_{2A}$ cDNA to the SV40 early promoter. A plasmid pSVIFA2-II was first constructed by subcloning the 5' Xho$_1$-BamH$_1$ (Xh-B) segment of the IFA-2 gene (from 5.5 kb to 7.5 kb in Figure 2) fused with a Cla$_1$-Xho$_1$ synthetic adaptor of 26 bp (restoring the 5' cDNA sequence), into a Cla$_1$ and Bam H$_1$-cut pBR plasmid. The 3' BamH$_1$-Hind$_{III}$ (B-H) gene segment (from 7.5 kb to 10.5 kb in Fire 2), subcloned in a a Hind$_{III}$, BamH$_1$-cut pBR plasmid, was excised using the Cla$_1$ site adjacent to Hind$_{III}$ in pBR322 and ligated to the above 5' segment to obtain the complete IFA-2 gene. A pSVE3 vector cut by Hind$_{III}$ as described in Y. Chernajovsky et al. (1984) <u>DNA</u> 3: 294-308, was reclosed with: Cla$_1$ linkers(forming pSVC1a) and the above IFA-2 gene introduced in the Cla$_1$ site of pSVC1a in the same orientation as the SV40 early promoter forming pSVIFA2-II. A plasmid pSVIFA2-I was similarly constructed but the Xho$_1$ site of IFA-2 was fused directly to the Cla$_1$ site of pBR before introduction in pSVE3. To obtain the pSVCIF$\beta$2 plasmid containing the IFN-$\beta_{2A}$ cDNA fused to the SV40 promoter, the above pSVIFA2-II DNA, cut by Xb$_1$, was partially cut by Xmn$_1$ to open the Xmn$_1$ site located 60 bp downstream from the Xho$_1$ site in the IFN-$\beta_2$ cDNA sequence (Figure 1), and not an Xmn$_1$ site located in the amp gene of the vector. To this construct, the Xmn$_1$-Xba$_1$ segment of IFN-$\beta_2$ cDNA clone AE20 (coordinates 92-566 in Fig. 1) was ligated, restoring the complete IFN-$\beta_{2A}$ cDNA sequence as shown in the Figure. EES is the early-early RNA start of SV40 T-ag gene; ATG is the IFN-$\beta_2$ initiation codon; pA are IFN-$\beta_2$ and SV40 polyadenylation sites, respectively. pSVCIF$\beta_2$ DNA was cotransfected with a pDHFR plasmid into CHO-K$_1$ DHFR$^-$ cells as in Y. Chernajovsky et al. (1984), (supra) and clones were tested for human IFN antiviral activity in FS11 cells using Vesicular Stomatitis Virus (VSV). Gene amplification was obtained by selecting CHO clones for methotrexate (MTX)-resistance. Assay of the culture medium of CHO-SVCIF$\beta_2$ B132-5M cells, resistant to 250 nM MTX, is shown in the lower part of the Figure. After VSV cytopathic effect developed, cells in the microplate were stained by methylene blue. Serial two-fold dilutions of the medium are from left to right. By comparison to IFN standard (ST) and non-transfected CHO cells, a titer of 300 U/ml rIFN-$\beta_2$ is calculated.

Figure 6 shows the results of an assay of (2'-5') oligo A synthetase with poly (rI)(rC)-agarose bound NP40 extracts (M. Revel et al. (1981) Meth. Enzymol. 79:149-161) of human FS11 fibroblasts previously exposed for 16 hours to medium from a) Cos7 cells two days post-transfection with the pSVIFA2-II DNA (described in Figure 5)(leftmost 4 lanes), b) hamster CHO SI-15 cells stably transformed by the pSVIFA2-I DNA of Fig. 5 (next 4 lanes), c) CFO MEIF-5 cells expressing IFN-$\beta_1$ gene (Y. Chernajovsky et al. (1984), supra) (next 2 lanes); d) CHO cells with DHFR gene only (next lane); e) fresh medium (Non Ind.). The IFN antiviral activity measured per ml of the media added to FS11 cell is indicated. An electrophoresis of phosphatased, 32P-$\alpha$-ATP labelled (2'-5') oligo A product is shown.

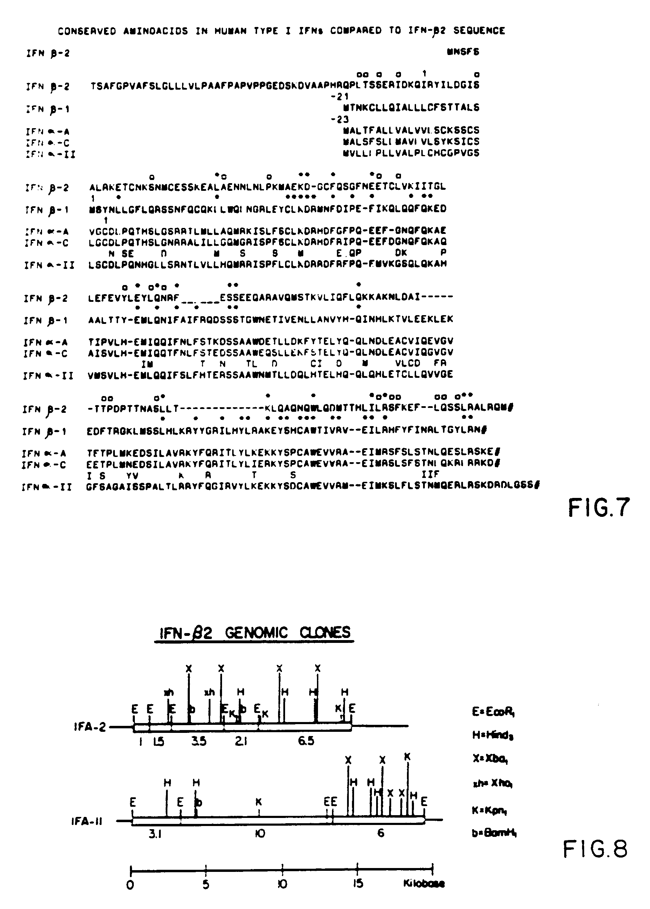

Figure 7 is a comparison of amino acid sequences between type I human IFNs. Amino acids that are conserved in IFN-$\beta_1$ and the various IFN-$\alpha$s (shown are $\alpha$A, $\alpha$C, replacements in other $\alpha$s excluding $\alpha$E, and IFN-$\alpha$ class II), are marked by stars over the IFN-$\beta_1$ sequence. Amino acids conserved in IFN-$\beta_2$ are shown above its sequence by stars if conserved in all type I IFNs, or by squares if conserved in some IFNs. IFN-$\beta_{2A}$ was aligned by comparing hydropathy plots (see text) and numbered from the presumed processing site.

Figure 8 shows restriction maps of the two different human IFN-$\beta_2$ genes which are expressed in rodent cells. The IFA-11 DNA (IFN-$\beta_{2B}$ gene) was transfected into either CHO or L cells and expressed IFN-$\beta$ activity after induction (Table 1).

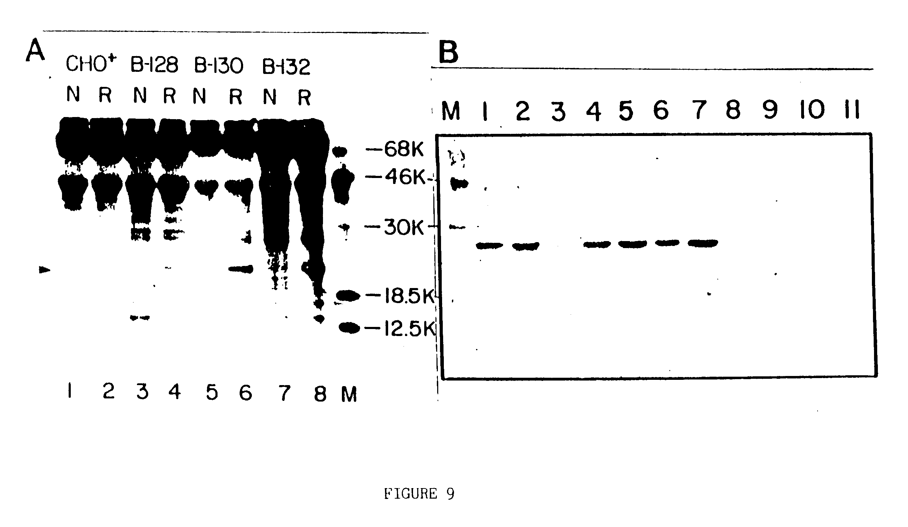

Figure 9 shows an IFN-$\beta_2$ specific immunocompetition assay with R-antibodies to show that human fibroblasts FS11 cells secrete the IFN-B$_2$ protein in response to the two cytokines interleukin-I (Il-I)and Tumor Necrosis Factor (TNF-$\alpha$). Both cytokines induce the synthesis and secretion of the IFN-$\beta_2$ protein in two levels which have been estimated by the immunoassay to be 10 to 20 U/ml IFN-$\beta_2$. Optimal IFN-$\beta_2$ induction was seen with the concentrations of rIL-1$\alpha$ of 4 U/ml (0.13 ng/ml). The optimal concentration of TNF-$\alpha$ for IFN-$\beta_2$ induction (400 U/ml; 40 ng/ml) was higher than that required for cytolytic effect on sensitive cells (0.1 ng/ml) and even higher than that giving optimal growth stimulation of diploid fibroblasts (2 ng/ml).

## Detailed description of preferred embodiments

The existence of interferon-beta$_2$ was discovered in the course of attempts to isolate genetic material containing the nucleotide sequence coding for interferon in human fibroblast cells. In this process, fibroblast cells were induced for the production of inducible interferon mRNA with a suitable exogenous factor and then the mRNAs were extracted. At the same time the mRNAs from a non-induced culture of the same host cells were extracted. cDNA probes were synthesized from the mRNAs of both the induced culture and the non-induced control culture using the corresponding mRNAs as templates (induced cDNAs and non-induced cDNAs). Double-stranded cDNAs derived from the mRNA extracted from the induced culture were then synthesized and such cDNAs were inserted in appropriate vectors and transfected into suitable microorganisms. The microorganisms were cultivated under conditions suitable to cause selective development of microorganism colonies of the modified vectors (initial colonies). Duplicate colonies of the initial colonies were formed and the DNAs of both the initial and duplicate colonies were freed in situ. The DNAs of one of the two sets of duplicate colonies were hybridized with the cDNA probes synthesized from the mRNAs extracted from the induced culture and the other of the two sets of duplicate colonies were hybridized with the cDNA probes synthesized from the mRNAs extracted from the non-induced culture. Clones which selectively hybridized with the induced cDNA probes but not with the non-induced cDNA probes were selected and their DNAs recovered. These cloned DNAs were further tested to determine those DNAs capable of hybridizing with mRNA translatable in frog oocytes or reticulocyte-lysates into interferon, which DNAs are essentially those which code for interferon, or contain enough of the sequence coding for interferon to be used to obtain a complete interferon DNA.

During this investigation, it was discovered that two different mRNAs coding for interferon could be isolated from human fibroblast cells after being appropriately induced. Upon glycerol gradient centrifugation, the smallest mRNA sediments at 11S and yields by translation in a cell-free system a protein of molecular weight 20,000 which is selectively precipitated by antibodies prepared against one of the interferons that can be purified from these cells. This protein is human interferon-beta$_1$ of which the amino acid sequence was partially determined by E. Knignt, et al. (1980) Science, 207, 525-526. The largest mRNA was found to sediment at 14S and yield a protein of molecular weight 23,000 which is precipitated by antibodies against a less purified preparation of fibroblast interferon. This protein is designated human interferon-beta$_2$. cDNA

clones hybridizable to mRNA which translates to interferon-beta$_2$ were prepared in such a manner and an IFN-$\beta_2$ cDNA probe was prepared therefrom.

Two such IFN-$\beta_2$ clones A341 ad E474 were found to represent over-lapping sequencing and were fused to reconstitute the IFN-$\beta_{2A}$ AE20 cDNA (Figure 1). The nucleotide sequence of this IFN-$\beta_{2A}$ cDNA was determined and revealed an open reading frame of 212 amino acids predicting a protein of 23,500 daltons. Transcription-translation experiments confirmed that this cDNA encodes such a protein.

From a human gene library of human adult blood cell DNA cloned after partial EcoRI digestion in λ charon 4A (Y. Mory et al. (1981) Eur. J. Biochem. 120: 197-202), two genomic clones IFA-2 and IFA-11 hybridizing to such an IFN-$\beta_2$ cDNA probe were isolated. Clone IFA-2 contains a gene with at least 4 exons hybridizing to various segments of the cloned cDNA (Figure 1). An Xho1 site present near the 5' of the AE20 cDNA allowed to map the genomic segment A132 (Figure 2) which contains the first IFA-2 exon ending 70 bp downstream from this Xho1 site . By S1 nuclease analysis using DNA probes labelled at a BstN1 site 40 bp after the Xho1 site, it was determined that induced FS11 human fibroblasts contain IFN-$\beta_{2A}$ RNA with two distinct 5' ends S-1 and S-2 separated by 20 nucleotides (Figure 3). Each of these two starts is preceded in the IFA-2 gene by a potential TATA box at -30 (Figure 4). RNA ending at S-2 seems to be more abundant than the longer RNA under a variety of induction conditions (Figure 3). Both RNA 5' ends were also seen with cytoplasmic RNA instead of total cell RNA. Therefore, it is apparent that both RNAs are formed and active in human cells. Both IFN-$\beta_{2A}$ RNAs are induced in human fibroblasts by poly (rI)(rC) and induction is stimulated by IFN priming as in the case of IFN-$\beta_1$ RNA in these cells (U. Nir et al. (1984) Nucl. Acids Res. 12: 6979-6993). A prolonged (6.5 h) treatement with CHX also induces IFN-$\beta_{2A}$ RNA, but shorter (3.5 h) CHX treatment is effective only with poly (rI)(rC) (Figure 3). The low induction of IFN-$\beta_{2A}$ RNA by CHX without ds RNA distinguishes this gene from IFN-$\beta_1$.

DNA constructs were made to express the IFA-2 gene under the control of the SV40 early gene promoter. A 4.8 kb segment of IFA-2 DNA was fused through a synthetic oligonucleotide to pSVE3 DNA (Y. Chernajovsky et al. (1984). supra) so that the ATG codon is 150 bp downstream from the SV40 early early RNA start (pSVIFA2-II, see legend, Figure 5). After transfection of pSVIFA2-II DNA into Cos7 monkey cells (Y. Gluzman (1981) Cell 23: 175-182), two RNAs of 1.35 and 2.2 kb were detected in Northern blots by IFN-$\beta_2$ cDNA; these two RNAs would correspond to transcripts ending at the IFN-$\beta_2$ and SV40 polyadenylation sites respectively (Figure 5). Medium from the Cos7 cells during transient expression of the pSVIFA2-II DNA was assayed for IFN activity by inhibition of the cytopathic effect of VSV on human FS11 and Wish cells as described in D. Novick et al. (1983) J.Gen.Virol. 64: 905-910. Antiviral activity could be clearly detected two days after transfection (Table 1). The specific antiviral activity of IFN-$\beta_{2A}$ per unit protein has been estimated to be 30 to 100 times lower than that of IFN-$\beta_1$ and closer to that of IFN-$\alpha_1$ (5-10 x $10^6$ units/mg). The IFN titers observed by transient expression of the IFA-2 gene were as expected, about 3% of those seen with the IFN-$\beta_1$ gene under the same SV40 promoter control (Table 1). Production of IFN-$\beta_{2A}$ activity by pSVIFA2-II DNA cells was further demonstrated by induction of (2'-5') oligo A synthetase (M. Revel et al. (1981), supra) in FS11 cells exposed to the medium of the transfected Cos7 cells (Figure 6). The induction was dose-dependent and corresponded quantitatively to the antiviral activity.

The IFA-2 gene was also fused to the SV40 early promoter directly at the Xho1 site near S-2 (pSVIFA2-I DNA, legend of Figure 5). Hamster CHO-K1 DHFR⁻ cells were cotransformed with pSVIFA2-I and pSVDHFR (Y. Chernajovsky et al. (1984), supra) DNAs and a stably transformed line SI-15 was isolated. This cell line produces constitutively 10-50 U/ml of IFN antiviral activity measured on human FS11 and Wish cells, while CHO cells transformed only by pSVDHFR produce no human IFN (Table 1). By concentration of the medium from SI-15 cultures, rIFN-$\beta_{2A}$ solutions titrating about 3000 U/ml could regularly be obtained. This rIFN-$\beta_{2A}$ has the properties expected for a human IFN and induces the (2'-5') oligo A synthetase in FS11 cells even at low concentrations of 1 U/ml (Figure 6). The rIFN-$\beta_{2A}$ constitutively produced by CHO SI-15 cells demonstrated human-specific induction of the mRNAs for (2'-5') oligo A synthetase, C56 and HLA in the presence of cycloheximide, its antiviral activity was neutralized by anti-IFN-$\beta_1$ (but not anti-IFN-$\alpha$ or anti-IFN -$\gamma$ ) polyclonal and monoclonal antibodies and it acted on mouse-human hybrids only if they contain human chromosome 21 which carries the gene for the type 1 IFN receptor. These further characterizations of the biological activity of rIFN-$\beta_{2A}$ convincingly demonstrate that it acts directly as a IFN and is not an inducer of IFN-$\beta_1$.

Improved yields of rIFN-$\beta_{2A}$ were obtained with DNA constructs containing the IFN-$\beta_{2A}$ cDNA sequence fused to the SV40 early promoter (pSVCIF$\beta$2 shown in Figure 5), when this DNA cotransfected with pSVDHFR into CHO cells as above, was amplified by selecting cells resistant to increasing concentrations of methotrexate. Without amplification, the pSVCIF$\beta_{2A}$ transformed CHO cells (e.g. clone B-132) produced levels of IFN-$\beta_{2A}$ activity similar to those produced by the SI-15 cells (Table 2). However, after selection for resistance to methotrexate, higher yields were obtained with up to 800 U/ml (Table 2).

From such clones it was possible to immunoprecipitate the IFN-$\beta_{2A}$ protein secreted into the culture medium and if the cells had been labelled by $^{35}$S-methionine to analyse the IFN-$\beta_{2A}$ protein by dodecyl sulphate polyacrylamide gel electrophoresis. The size of the secreted rIFN-$\beta_{2A}$ was found to be 21,000 daltons. Indeed, experiments have shown that the 23-26 Kd primary translation product of IFN-$\beta_{2A}$ RNA in reticulocyte lysates is processed in vitro by dog pancreatic membranes into a shortened 21 Kd protein, and that induced human fibroblasts produce a 21 Kd protein which is immunologically similar to the IFN-$\beta_{2A}$ RNA 26 Kd product, but Distinct from IFN-$\beta_1$. The N-terminus of the mature IFN-$\beta_{2A}$ 21 Kd protein has not been determined and two potential glycosylation sites are present in the IFN-$\beta_{2A}$ sequence (Figure 1) making it difficult to calculate the size of the region removed by processing. However, the decrease in size indicates that this processed region is longer than that in IFN-$\beta_1$, the size of which increases by maturation (W. Degrave et al. (1981) Gene 10: 11-15). A hydropathy plot of the IFN-$\beta_{2A}$ 26 Kd polypeptide has shown that there is a marked similarity between hydrophobic and hydrophilic regions of IFN-$\beta_{2A}$ and of mature IFN-$\beta_1$, when the two proteins are aligned by their C-termini. The same alignment also reveals conservation in the amino acid sequence of IFN-$\beta_{2A}$ with those of the other type I human IFNs (Figure 7). Between all the known IFN-$\alpha$s and $\beta_1$ sequences, there are 38 amino acids which are conserved (marked by stars in Figure 7). When IFN-$\beta_{2A}$ is aligned as above, 18 of these 38 amino acids common to all type I human IFNs can be seen to be conserved (Figure 7). There is an overall homology of about 20% between IFN-$\beta_{2A}$ and the other type I IFNs.

Although the sequence homology between IFN-$\beta_1$ and $\beta_{2A}$ is low, the cross-neutralization of the antiviral activities of IFN-$\beta_1$ and IFN-$\beta_{2A}$ by the same antibodies, including a monoclonal anti-IFN-$\beta_1$ (D. Novick et al. (1983), supra), could result from some conserved structure in the active site of IFN-$\beta$. IFN-$\beta_{2A}$ activity is not neutralized by anti-IFN-$\alpha$ or $\gamma$ antibodies, justifying its designation as a $\beta$-type IFN. The genes encoding IFN-$\beta_2$ are not on chromosome 9 (U. Nir (1984), supra) and differ from the type I IFN genes in the chromosome 9 cluster by the presence of introns. The IFA-2 gene was determined to be on chromosome 7 (P.B. Sehgal et al. (1986) Proc.Natl.Acad.Sci. USA, in press). The amino acid sequence homology detected is compatible with the notion that the IFN-$\beta_2$ genes are related to the ancestral gene which gave rise to the type I IFN gene cluster.

The second genomic DNA clone IFA-11 hybridizing with the IFN-$\beta_{2A}$ cDNA has a restriction map which differs from that of IFA-2 (Figure 8). Transcript mapping and sequencing showed that the 3' exon of IFA-2 and IFA-11 (XbaI-HindIII segment) are identical (99% sequence homology), while there was no cross-hybridization under stringent conditions between more 5' exons. The IFA-11 genomic clone (19 kb) was transfected into L-TK$^-$ cells together with a HSV-TK gene (F. Colbere-Garapin et al. (1981) J.Mol.Biol. 150: 1-14) and stable transformants were isolated. Table 1 shows that L cell clone LI-39 containing the human IFA-11 gene, produces human IFN activity when induced by poly (rI)(rC) and cycloheximide, while L-TK$^+$ cells produced no such activity. A CHO clone 1C40 containing the IFA-11 gene also produced human IFN activity (Table 1). No activity was seen in non-induced LI-39 or 1C40 cells. The IFN activity obtained with IFA-11 transformants after induction was significantly higher than with the IFA-2 gene. The rIFN-$\beta_{2B}$ produced by expression of the IFA-11 gene was neutralized with anti-IFN-$\beta_1$ similarly to the IFA-2 gene product. IFA-11 specific DNA probes hybridized to RNA from induced human fibroblasts similarly to IFN-2 DNA probes, indicating that both genes are active in human cells.

The function of the IFN-$\beta_2$ genes seems to result from their expression under conditions in which other IFN genes are not expressed. We find that fibroblasts exposed to Tumor Necrosis Factor (TNF) or to lymphokine IL-1 produce the IFN-$\beta_2$ protein. Others found that neutralization of IFN-$\beta$ by antibodies results in a stimulation of cell proliferation (M. Kohase et al. (1986) Cell, in press; D. Resnitzky et al. (1986) Cell, in press), suggesting that the IFN-$\beta_2$ acts to limit cell growth in response to growth factors. Supporting this conclusion is the fact that RNA hybridizing to IFN-$\beta_2$ cDNA was recently detected in peripheral blood mononuclear cells induced by mitogens such as PHA along with IFN-$\gamma$ mRNA. It has also been reported that an IL-1 like lymphokine strongly induces RNA hybridizing to IFN-$\beta_2$ cDNA in diploid fibroblasts and other human cell lines (J. Content et al. (1985) Eur.J.Biochem. 152: 253-257). The promoter region of the IFA-2 genes seems to contain two TATA boxes and two RNA starts (Figures 1 and 3). This IFN-$\beta_2$ promoter was shown to respond to either poly (rI)(rC) or to cycloheximide (Y. Chernajovsky et al. (1984), supra). In vivo, the two IFN-$\beta_2$ gene promoters may be activated by different inducers (ds RNA, protein synthesis inhibitors, IL-1, PHA). A variety of cells have been found to produce autocrine IFN-$\beta$ in low amounts during growth arrest and differentiation (M. Revel (1983) Interferon 5 pp.205-239, Acad. Press, London). IFN-$\beta_2$ may belong to the same group of minor IFN species produced by cells to auto-regulate their growth, while the classical type I IFNs are probably more specifically involved in the response to viral infections.

The biological significance of IFN-$\beta_2$ lies most probably in the fact that it is induced under conditions where IFN-$\beta_1$ is not induced, as in metabolically stressed cells (A. Zilberstein et al., (1985) in The Interferon System, Serono Symposia Raven Press, pp.73-83). Most important is the observation that TNF Induces IFN-$\beta_2$ in fibroblasts and that the proliferation of these cells which is stimulated by TNF is further enhanced if anti-IFN-$\beta$ antibodies are added. A number of human cell lines have been shown to produce autocrine IFN-$\beta$ species when undergoing differentiation, and these IFNs are responsible for the induction of HLA antigens on these cells (A. Yarden et al. (1984) Embo.J. 3: 969-973). In a mouse myeloleukemic cell line induced to differentiate by CSF-1, the growth arrest of the cells which characterizes terminal differentiation, is abolished by anti-IFN-$\beta$ antibodies. IL-1 stimulates growth of several cell types and the classical growth factor PDGF was found to induce IFN-$\beta$ RNA in mouse cells (J.N. Zullo et al. (1985) Cell 43: 793-800). Although multiple IFN-$\beta$ species may be involved (P.B. Sehgal, (1982) in Interferon 4, Academic Press, pp.1-22, and M. Revel (1983) in Interferon 5, Academic Press, pp. 205 - 239), IFN-$\beta_2$ appears to be one of these autocrine autoregulators of cell growth produced in response to growth factors. This would be in line with the low specific activity of IFN-$\beta_2$ (estimated by comparison of immunoassay and antiviral activity to be 50-100 times lower than for IFN-$\beta_1$), since only very small antiviral activity is seen in cells producing the autocrine IFN-$\beta$ species (M. Revel (1983) in Interferon 5, Academic Press, pp. 205-239). This could also explain why the hamster cells expressing IFN-$\beta_2$ cDNA produce less IFN activity than similar CHO cells harbouring the human IFN-$\beta_1$ gene (Y. Chernajovsky et al. (1984), DNA 3: 297-308). Interestingly, the IFN-$\alpha$ 1 (D) species has also a 100 times lower specific activity on human cells than other IFN-$\alpha$ species (T. Goren et al. (1983), Virology 130: 273-280), although it represents a large proportion of the total mass of leukocyte IFN (C. Weissmann (1981) in Interferon 3, Academic Press, N.Y. pp.101-134). It is not excluded that some of the multiple IFN functions will be expressed more efficiently by IFN-$\beta_2$ than the antiviral effect. Despite their law concentration, growth-regulatory IFN-$\beta$ Induce HLA and (2'-5') oligo A synthetase more strongly than expected, have more prolonged effects and markedly inhibit the growth of the cells which produce them. The induction of IFN-$\beta_2$ by IL-1 and TNF suggests that it may play a role as an autocrine mediator of some effects of these cytokines in inflammation and acute-phase responses, as well as regulating cell proliferation.

Induction of mRNA which hybridizes with IFN-$\beta_2$ cDNA probes but not to IFN-$\beta_1$ cDNA has been observed in human cell lines after exposure to Interleukin-1 (IL-1) (J. Content et al. (1985), Eur.J.Biochem. 152: 253-257) and Tumor Necrosis Factor (TNF-$\alpha$). The IFN-$\beta_2$-specific immunocompetition assay with R-antibodies was used to examine if human fibroblasts FS11 cells indeed secrete the IFN-$\beta_2$ protein in response to these two cytokines (Figure 9). Both rIL-1$\alpha$ and TNF-$\alpha$ induced the synthesis and secretion of the IFN-$\beta_2$ protein, to levels estimated by the immunoassay to be 10-20 U/ml IFN-$\beta_2$. Optimal IFN-$\beta_2$ induction was seen with concentrations of rIL-1$\alpha$ of 4 U/ml (0.13 ng/ml), similar to those required for IL-1 induction of prostaglandin E$_2$, collagenase and hyaluronic acid in human fibroblasts (J.H. Korn (1985), Arthritis Rheum. 28: 315-322). The optimal concentration of TNF-$\alpha$ for IFN-$\beta_2$ induction (400 U/ml; 40 ng/ml) was higher than that required for the cytolytic effect on sensitive cells (0.1 ng/ml; A.M. Wang et al. (1985) Science 228: 149-154) and even higher than that giving optimal growth stimulation of diploid fibroblasts (2 ng/ml). The onset of IFN-$\beta_2$ secretion was somewhat faster with TNF-$\alpha$ reaching half maximal levels at about 6 hours, versus 8-12 hours with IL-1.

To the extent that it may be demed necessary in order to complete the disclosure in this case, all of the subject matter of each of the publications referred to and cited hereinabove are hereby incorporated herein by reference.

It will be obvious to those skilled in the art that various other changes and modifications may be made without departing from the scope of the invention and the invention is not to be considered limited to what is shown in the drawings and described in the specification.

## TABLE 1

Antiviral Activity of rIFN-$\beta_2$* Produced in Transfected Mammalian Cells

I.   IFA-2 GENE

A.   <u>Constitutive expression in CHO cells</u>

pSVIFA2-I                S1-15 cells        :    10-50 U/ml**

(control                 CHO cells          :    0                )

B.   <u>Transient expression in Cos7 cells</u>

pSVIFA2-II               DNA                :    15 U/ml

(control IFN-$\beta_1$   pSVEIF DNA         :    500 U/ml)

II.  IFA-11 GENE

A.   <u>Induced expression*** in CHO cells</u>

Clone 1C40                                  :    400-500 U/ml

(control                 CHO cells          :    100 U/ml)

B.   <u>Induced expression in L cells</u>

Clone LI-39                                 :    250 U/ml

(control L-TK$^+$ cells                     :    < 4 U/ml)


*     Assay with VSV in FS11 and WISH cells.
      Neutralized by anti-IFN-$\beta_1$.

**    3000 U/ml after concentration.

***   Induction with poly IC and cycloheximide for 10 hrs.
      Harvest at 28 hrs.

TABLE 2

| Antiviral Activity of Human rIFN-$\beta_{2A}$ Produced in Hamster Cells Transfected and Amplified with IFN-$\beta_{2A}$ cDNA | | |
|---|---|---|
| CHO-SVCIF$\beta$2 B132-10M | CLONE: | Human IFN activity* U/ml |
| B-132 | (0 MTX)** | 64 |
| B-132-5M | (250 nM MTX)** | 300 |
| B-132-10M | (500 nM MTX)** | 800 |

* Medium from cultures was collected every 24 hours and assayed with VSV in FS11 human cells. The activity was neutralized by anti-IFN-$\beta_1$, but not anti-IFN-$\alpha$ or anti-IFN-$\gamma$.
** Cultures were selected for resistance to indicated concentrations of methotrexate.

**Claims**

1. A DNA consisting essentially of a nucleotide sequence encoding the amino acid sequence of a biologically active interferon-$\beta_{2A}$ shown in SEQ ID No. 1, wherein said amino acid sequence has the N-terminus of mature interferon-$\beta_{2A}$.

2. A DNA capable of hybridizing to a DNA according to claim 1 and which is capable of expressing a biologically active human interferon-$\beta_{2A}$.

3. A DNA according to claim 1 or 2 which is a cDNA.

4. A recombinant vector comprising a DNA sequence according to claims 1, 2 or 3 and further comprising regulatory regions which are positioned in such a way that expression of a biologically active human interferon-$\beta_{2A}$ is possible.

5. A host cell transformed by a recombinant vector according to claim 4 which is capable of producing a biologically active human interferon-$\beta_{2A}$.

6. A host cell according to claim 5, wherein the host cell is a prokaryotic host cell.

7. A process for producing a biologically active human interferon-$\beta_{2A}$, wherein a host cell according to claim 5 or 6 is cultivated and the interferon-$\beta_{2A}$ is recovered.

8. A recombinant human biologically active interferon-$\beta_{2A}$ in purified form having the amino acid sequence shown in SEQ ID No. 1, wherein said amino acid sequence has the N-terminus of mature interferon-$\beta_{2A}$.

9. A pharmaceutical composition comprising a recombinant human biologically active interferon-$\beta_{2A}$ according to claim 8.

IFN-β2a cDNA

0    250    500    750    1000    1250

SI S2    ATG    TAG

(A132)

AE20 { E474
A36 }

AhaⅢ AluI BstNI HinfI PstI Sau3a TaqI XhoI XmnI XbaI HpaⅡ

IFN-Beta 2a cDNA

```
            10              20              30              40              50              60
             •               •               •  - XhoI -    •               •               •
AG GAC TGG AGA TGT CTG AGG CTC ATT CTG CCC TCG AGC CCA CCG GGA ACG AAA GAG AAG CTC
            70              80              90             100             110             120
             •               •               •  - XmnI -   •               •               •
TAT CTC CCC TCC AGG AGC CCA GCT ATG AAC TCC TTC TCC ACA AGC GCC TTC GGT CCA GTT
                                        Met Asn Ser Phe Ser Thr Ser Ala Phe Gly Pro Val
                                         1                                              10
           130             140             150             160             170             180
             •               •               •               •               •               •
GCC TTC TCC CTG GGG CTG CTC CTG GTG TTG CCT GCT GCC TTC CCT GCC CCA GTA CCC CCA
Ala Phe Ser Leu Gly Leu Leu Leu Val Leu Pro Ala Ala Phe Pro Ala Pro Val Pro Pro
                          20                                              30
           190             200             210             220             230             240
             •               •               •               •               •               •
GGA GAA GAT TCC AAA GAT GTA GCC GCC CCA CAC AGA CAG CCA CTC ACC TCT TCA GAA CGA
Gly Glu Asp Ser Lys Asp Val Ala Ala Pro His Arg Gln Pro Leu Thr Ser Ser Glu Arg
                          40                                              50
           250             260             270             280             290             300
             •               •               •               •               •               •
ATT GAC AAA CAA ATT CGG TAC ATC CTC GAC GGC ATC TCA GCC CTG AGA AAG GAG ACA TGT
Ile Asp Lys Gln Ile Arg Tyr Ile Leu Asp Gly Ile Ser Ala Leu Arg Lys Glu Thr Cys
                                      60                                      70
           310             320             330             340             350             360
             •               •               •               •               •               •
AAC AAG AGT AAC ATG TGT GAA AGC AGC AAA GAG GCA CTG GCA GAA AAC AAC CTG AAC CTT
Asn Lys Ser Asn Met Cys Glu Ser Ser Lys Glu Ala Leu Ala Glu Asn Asn Leu Asn Leu
                                      80                                      90
           370             380             390             400             410             420
             •               •               •               •               •               •
CCA AAG ATG GCT GAA AAA GAT GGA TGC TTC CAA TCT GGA TTC AAT GAG GAG ACT TGC CTG
Pro Lys Met Ala Glu Lys Asp Gly Cys Phe Gln Ser Gly Phe Asn Glu Glu Thr Cys Leu
                                      100                                     110
           430             440             450             460             470             480
             •               •               •               •               •               •
GTG AAA ATC ATC ACT GGT CTT TTG GAG TTT GAG GTA TAC CTA GAG TAC CTC CAG AAC AGA
Val Lys Ile Ile Thr Gly Leu Leu Glu Phe Glu Val Tyr Leu Glu Tyr Leu Gln Asn Arg
                                      120                                     130
           490             500             510             520             530             540
             •               •               •               •               •               •
TTT GAG AGT AGT GAG GAA CAA GCC AGA GCT GTC CAG ATG AGT ACA AAA GTC CTG ATC CAG
Phe Glu Ser Ser Glu Glu Gln Ala Arg Ala Val Gln Met Ser Thr Lys Val Leu Ile Gln
                                      140                                     150
           550             560             570             580             590             600
             •               •               •  -XbaI• -    •               •               •
TTC CTG CAG AAA AAG GCA AAG AAT CTA GAT GCA ATA ACC ACC CCT GAC CCA ACC ACA AAT
Phe Leu Gln Lys Lys Ala Lys Asn Leu Asp Ala Ile Thr Thr Pro Asp Pro Thr Thr Asn
                                      160                                     170
           610             620             630             640             650             660
             •               •               •               •               •               •
GCC AGC CTG CTG ACG AAG CTG CAG GCA CAG AAC CAG TGG CTG CAG GAC ATG ACA ACT CAT
Ala Ser Leu Leu Thr Lys Leu Gln Ala Gln Asn Gln Trp Leu Gln Asp Met Thr Thr His
                                      180                                     190
           670             680             690             700             710             720
             •               •               •               •               •               •
CTC ATT CTG CGC AGC TTT AAG GAG TTC CTG CAG TCC AGC CTG AGG GCT CTT CGG CAA ATG
Leu Ile Leu Arg Ser Phe Lys Glu Phe Leu Gln Ser Ser Leu Arg Ala Leu Arg Gln Met
                                      200                                     210
           730             740             750             760             770             780
             •               •               •               •               •               •
TAG CAT GGG CAC CTC AGA TTG TTG TTG TTA ATG GGC ATT CCT TCT TCT GGT CAG AAA CCT
           790             800             810             820             830             840
             •               •               •               •               •               •
GTC CAC TGG GCA CAG AAC TTA TGT TGT TCT CTA TGG AGA ACT AAA AGT ATG AGC GTT AGG
           850             860             870             880             890             900
             •               •               •               •  -Aha3 •-     •               •
ACA CTA TTT TAA TTA TTT TTA ATT TAT TAA TAT TTA AAT ATG TGA AGC TGA GTT AAT TTA
           910             920             930             940             950             960
             •               •               •               •               •               •
TGT AAG TCA TAT TTT ATA TTT TTA AGA AGT ACC ACT TGA AAC ATT TTA TGT ATT AGT TTT
           970             980             990            1000            1010            1020
             •               •               •               •               •               •
GAA ATA ATA ATG GAA AGT GGC TAT GCA GTT TGA ATA TCC TTT GTT TCA GAG CCA GAT CAT
          1030            1040            1050            1060            1070            1080
             •               •               •               •               •  -Aha3  •
TTC TTG GAA AGT GTA GGC TTA CCT CAA ATA AAT GGC TAA CTT TAT ACA TAT TTT TAA AGA
          1090            1100            1110            1120            1130            1140
             •               •               •               •               •               •
AAT ATT TAT ATT GTA TTT ATA TAA TGT ATA AAT GGT TTT TAT ACC AAT AAA TGG CAT TTTAAA...
```

FIG.1

STRUCTURE OF HUMAN IFNβ2 GENE IFA-2

FIG.2

FIG.3

IFN-β2 RNA  FS11 CELLS

```
           -74                        -51
TAAATATAGGACTGGAGATGTCTGAGGCTCATTCTGCCCTCGAG......BstnI
        S-1                   ***    S-2
```

IFN-β2 Gene, Clone IFA-2

```
        -150      -140      -130      -120      -110      -100
IFA-2   ACTAAAGGACGTCACATTGCACAATCTTAATAAGGTTTCCAATCAGCCCCACCCGCTCTG

        -90       -80       -70       -60       -50       -40
IFA-2   GCCCCACCCTCACCCTCCAACAAAGATTTATCAAATGTGGGATTTTCCCATGAGTCTCAA

        -30       -20       -10        1        10        20
IFA-2   TATTAGAGTCTCAACCCCCAATAAATATAGGACTGGAGATGTCTGAGGCTCATTCTGCCC
cDNA    *****              ******     -------...----------ATTCTGCCC
        TATA-1              TATA-2    S-1                  S-2

        30xhoI    40        50        60        70        80
IFA-2   TCGAGCCCACCGGGA-CGAAAGAGAAGCTCGATCTCCCCTCCAGGAGCCCAGCTATGAAC
cDNA    TCGAGCCCACCGGGAACGAAAGAGAAGCTCTATCTCCCCTCCAGGAGCCCAGCTATGAAC
                                                              ✗

        90        100  intr1 110      120       130       140
IFA-2   TCCTTCTCCACAA GTAAGTGCAGGAAATCCTTAGCCCTGAACTGCCAGCGGGGTCGAGCC
cDNA    TCCTTCTCCACAA GCGCCTTCGCTCCAGTTGCCTTCTCCCTGGGGCTGCTC....

        150       160       170       180       190       200
IFA-2   CTGTGTGAGGGAGGGGTGTGTGGCCCAAGGAGGGCTGGCGGGGCGGCCAGCAGCAGAGGCA

        210       220       230       240       250
IFA-2   GGCTCCCAGCTGTGCTGTCAGCTCACCCTGCGCTCGCTCCCTCGC....
```

FIG.4

CONSTITUVE EXPRESSION OF Hu IFN β2 ÍN CHO CELLS

FIG.5

clone B132 250nM MMTX = 300 UNITS/ ML   (VSV, Mengo)

INDUCTION OF 2'-5' OLÍGO A SYNTHETASE BY
r-IFN-β2 FROM GENE TRANSFECTED CELLS

FIG.6

| PRODUCT OF pSVIFA2-II in COS7 cells | | | | PRODUCT OF pSVIFA2-I in CHO cells antiviral units | | | | IFN-β1 CHO cells | | CHO cell controls | NON IND. |
|---|---|---|---|---|---|---|---|---|---|---|---|
| 1 | 5 | 10 | 20 | 1 | 5 | 10 | 20 | 1 | 20 | 0 | 0 |

2'-5'ApApA —

2'-5' ApA —

CONSERVED AMINOACIDS IN HUMAN TYPE I IFNs COMPARED TO IFN-β2 SEQUENCE

```
IFN β-2                                                                    MNSFS

                                            oo  o   o    1       o
IFN β-2    TSAFGPVAFSLGLLLVLPAAFPAPVPPGEDSKDVAAPHRQPL TSSERIDKQIRYILDGIS
                                              -21
IFN β-1                                     MTNKCLLQIALLLCFSTTALS
                                              -23
IFN ∝-A                                     MALTFALLVALVVL SCKSSCS
IFN ∝-C                                     MALSFSLI MAVI VLSYKSICS
IFN ∝-II                                    MVLLI PLLVALPLCHCGPVGS


             o           •o       o      ••    •      •o  o
IFN β-2    ALAKETCNKSNMCESSKEALAENNLNLPKMAEKD-GCFQSGFNEETCLVKIITGL
           1    •           •          •••••   •   •   •     • ••
IFN β-1    MSYNLLGFLQRSSNFQCQKI LWQI NGRLEYCLKDRMNFDIPE-FIKQLQQFQKED
           1
IFN ∝-A    VGCDLPQTHSLGSRRTLMLLAQMRKISLFSCLKDRHDFGFPQ-EEF-GNQFQKAE
IFN ∝-C    LGCDLPQTHSLGNARALILLGQMGRISPFSCLKDRHDFRIPQ-EEFDGNQFQKAQ
             N SE    D        M   S   S  M    E GP      DK        P
IFN ∝-II   LSCDLPQNHGLLSRNTLVLLHQMRRISPFLCLKDRRDFRFPQ-FMVKGSQLQKAH


             o • o•o •          ••                 •
IFN β-2    LEFEVYLEYLQNRF___ ____ESSEEQARAVQMSTKVLIQFLQKKAKMLDAI-----
                  •  •   •  •      • ••                    •
IFN β-1    AALTTY-EMLQNIFAIFRQDSSSTGWNETIVENLLANVYH-QINMLKTVLEEKLEK

IFN ∝-A    TIPVLH-EMIQQIFNLFSTKDSSAAWDETLLDKFYTELYQ-QLNDLEACVIQEVGV
IFN ∝-C    AISVLH-EMIQQTFNLFSTEDSSAAWEQSLLEKFSTELYQ-QLNDLEACVIQGVGV
                      IM      T N    TL D      CI D   M    VLCD  FA
IFN ∝-II   VMSVLH-EMLQQIFSLFHTERSSAAWNMTLLDQLHTELHQ-QLQHLETCLLQVVGE


             oo      o•           •        •o•oo        oo o••
IFN β-2    -TTPDPTTNASLLT-------------KLQAQNQWLQDMTTHLILRSFKEF--LQSSLRALRQM/
             •     •    •  ••    • ••  •••    ••      •• •        ••
IFN β-1    EDFTRQKLMSSLHLKAYYGRILHYLRAKEYSHCAWTIVRV--EILRMFYFINRLTGYLRN/

IFN ∝-A    TFTPLMKEDSILAVRKYFQRITLYLKEKKYSPCAWEVVRA--EIMRSFSLSTNLQESLRSKE/
IFN ∝-C    EETPLMNEDSILAVRKYFQRITLYLIERKYSPCAWEVVRA--EIMRSLSFSTNI QKRI RRKD/
             I S    YV      K   R      T     S                   IIF
IFN ∝-II   GFSAGAISSPALTLRRYFQGIRVYLKEKKYSDCAWEVVAM--EIMKSLFLSTNMQERLRSKDRDLGSS/
```

FIG.7

IFN-β2 GENOMIC CLONES

E=EcoR₁
H=Hind₃
X=Xba₁
xh=Xho₁
K=Kpn₁
b=BamH₄

FIG.8

FIGURE 9

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| D,X | GB-A-2 063 882 (YEDA RESEARCH AND DEVELOPMENT COMPANY LIMITED) * the whole document * | 1-8 | C12N15/24 C07K14/54 C12N1/21 A61K38/20 |
| X | PROCEEDINGS OF THE NATIONAL ACADEMY OF SCIENCES OF USA, vol.82, August 1985, WASHINGTON US pages 5490 - 5494 TOSHIO HIRANO ET AL 'Purification to homogeneity and characterization of human B-cell differentiation factor (BCDF or BSFp-2)' | 1-8 | |
| D,X | PROCEEDINGS OF THE NATIONAL ACADEMY OF SCIENCES OF USA, vol.77, no.12, December 1980, WASHINGTON US pages 7152 - 7156 J. WEISSENBACH ET AL 'Two interferon mRNAs in human fibroblasts : In vitro translation and Escherichia coli cloning studies' * the whole document * | 1-8 | |
| P,X | EUROPEAN JOURNAL OF BIOCHEMISTRY, vol.159, no.3, 15 September 1986 pages 625 - 632 G. HAEGEMAN ET AL 'Structural analysis of the sequence coding for an inducible 26-kDa protein in human fibroblasts' * the whole document especially figure 3 * | 1-8 | |
| E | EP-A-0 257 406 (AJINOMOTO CO., INC.) * page 6 - page 10 * * page 11, line 49 - page 12 * * claims; examples 4-8 * | 1-8 | |

TECHNICAL FIELDS SEARCHED (Int.Cl.4)

C07K
A61K
C12N

-/--

The present search report has been drawn up for all claims

| Place of search | Date of completion of the search | Examiner |
|---|---|---|
| THE HAGUE | 2 February 1995 | Le Cornec, N |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| X | PATENT ABSTRACTS OF JAPAN vol. 10, no. 13 (C-323) (2070) 18 January 1986 & JP-A-60 169 424 (KISHIMOTO CHIYUUZOU ET AL) 2 September 1985 * abstract * --- | 1-9 | |
| E | PATENT ABSTRACTS OF JAPAN vol. 11, no. 96 (C-412) (2543) 26 March 1987 & JP-A-61 246 197 (AJINOMOTO CO ,INC.) 1 November 1986 * abstract * ----- | 1-9 | |
| | | | TECHNICAL FIELDS SEARCHED (Int.Cl.4) |

The present search report has been drawn up for all claims

| Place of search | Date of completion of the search | Examiner |
|---|---|---|
| THE HAGUE | 2 February 1995 | Le Cornec, N |

EPO FORM 1503 03.82 (P04C01)